# EUROPEAN PATENT APPLICATION

(11) **EP 4 835 487 A1**
(43) Date of publication of application: **04.11.2026**
(21) Application number: 24912686.3
(22) Date of filing: 20.12.2024
(51) Int. Cl.: G01N 35/10, G01N 37/00

(54) **SHEET-LIKE STRUCTURE**

(30) Priority: 25.12.2023 JP 2023217596
(71) Applicant: Dexerials Corporation, Shimotsuke-shi, Tochigi 323-0194 (JP)
(72) Inventor: HIRAKAWA, Manabu, Shimotsuke-shi, Tochigi 323-0194 (JP); MONJU, Takuya, Shimotsuke-shi, Tochigi 323-0194 (JP)
(74) Representative: Fleuchaus & Gallo Partnerschaft mbB
(86) International application number: PCT/JP2024/045321
(87) International publication number: WO 2025/142799

(57) **Abstract**

A sheet-like structure includes a porous structure layer including a flow path and a non-flow path. The flow path has a porous structure through which a fluid can pass, and the non-flow path does not allow the fluid to pass through. The flow path has a fluid receiving portion, a flow path portion, and a detection portion. The fluid receiving portion, the flow path portion, and the detection portion are connected in this order so that the fluid can pass through. A ratio (CFT_{d}/CFT_{f}) of capillary flow time CFT_{d} of the detection portion to capillary flow time CFT_{f} of the flow path portion is 1.2 or greater.

## Description

### TECHNICAL FIELD

The present invention relates to a sheet-like structure.

### BACKGROUND ART

Inspection devices that enable simple and rapid diagnosis in daily life or clinical practice have been developed. A representative example of an inspection device is a pregnancy test kit. When a test liquid including a target substance, such as an antigen, is introduced into the inspection device, the test liquid flows through a flow path within the inspection device. Then, a labeling medium such as an antibody, which has been disposed in the flow path in advance, reacts with the target substance included in the testing liquid to develop a color (to generate a color) so that the presence of the target substance can be confirmed.

Inspection chips, which are one example of the inspection devices, are also referred to as "µ-PADs (microfluidic paper-based analytical devices)." The inspection chips have many advantages, such as (1) low cost, (2) pumpless, (3) no need for complex devices, and (4) easy disposal, and therefore studies for improving the inspection chips have been conducted worldwide.

Various inspection chips (inspection devices) have been already reported. For example, an inspection chip in which a three-dimensional flow path is formed within a single sheet-like material is proposed for the purpose of effectively minimizing color development unevenness (see Patent Document 1). Further, for the purpose of easily producing an inspection chip at low cost, there is proposed a method in which the outer edge of the above-described flow path or reaction spot is printed on paper using an ultraviolet ray curable ink, and ultraviolet rays are applied to cure the ink, thereby easily producing an inspection chip at low cost (see Patent Document 2).

### CITATION LIST

### PATENT DOCUMENTS

Patent Document 1: Japanese Unexamined Patent Application Publication No. 2021-175970
Patent Document 2: International Publication No. WO 2012/160857

### SUMMARY OF THE INVENTION

### TECHNICAL PROBLEM

In the inspection chip of the related art disclosed in Patent Document 1, a flow rate of a fluid is controlled by the flow path design, such as a flow path width, but a flow rate of the fluid is high, and therefore the flow rate cannot be controlled by the change of the flow direction due to the stacked three-dimensional flow path. Thus, unevenness in color development cannot be sufficiently inhibited, and further improvement is desired. In addition, the inspection chip of the related art disclosed in Patent Document 2 tends to cause unevenness in color development (coloration), and may cause unevenness in inspection results (insufficient reproducibility). As one example, there is a problem that the coloration occurs in the vicinity of the edge of the detection area, and therefore it is difficult to visually observe the coloration.

The present invention aims to solve the above various problems in the related art, and to achieve the following object. That is, the present invention has an object to provide a sheet-like structure in which color development is evenly performed, and quantitative accuracy is improved, while maintaining rapidness of a reaction.

### SOLUTION TO THE PROBLEM

The means for solving the problems above are as follows.
<1> A sheet-like structure, including:
   a porous structure layer including a flow path and a non-flow path, the flow path having a porous structure through which a fluid can pass, and the non-flow path not allowing the fluid to pass through,
   wherein the flow path has a fluid receiving portion, a flow path portion, and a detection portion,
   the fluid receiving portion, the flow path portion, and the detection portion are connected in this order so that the fluid can pass through, and
   a ratio (CFT_{d}/CFT_{f}) of capillary flow time CFT_{d} of the detection portion to capillary flow time CFT_{f} of the flow path portion is 1.2 or greater.
<2> The sheet-like structure according to <1>,
   wherein the ratio (CFT_{d}/CFT_{f}) of the capillary flow time CFT_{d} of the detection portion to the capillary flow time CFT_{f} of the flow path portion is 1.2 or greater and 2.9 or less.
<3> The sheet-like structure according to <1> or <2>,
   wherein the capillary flow time CFT_{f} of the flow path portion is 120 sec/4 cm or less.
<4> The sheet-like structure according to any one of <1> to <3>,
   wherein the capillary flow time CFT_{d} of the detection portion is 300 sec/4 cm or less.
<5> The sheet-like structure according to any one of <1> to <4>,
   wherein the non-flow path is the porous structure impregnated with a hydrophobic material.
<6> The sheet-like structure according to any one of <1> to <5>,
   wherein the flow path includes the fluid receiving portion and the flow path portions, which communicate with a first surface and a second surface of the porous structure layer, and the detection portion that communicates with the first surface but is not exposed at the second surface.
<7> The sheet-like structure according to <6>,
   wherein the sheet-like structure further comprises a support layer on the second surface of the detection portion, or
   the porous structure at the second surface of the detection portion is impregnated with a hydrophobic material.
<8> The sheet-like structure according to <3> or <6>, further including:
   a support layer on the second surface of the fluid receiving portion and the flow path portion.
<9> The sheet-like structure according to <8>,
   wherein the support layer is impermeable to water.
<10> The sheet-like structure according to any one of <1> to <9>,
   wherein the sheet-like structure is an inspection device.

### EFFECTS OF THE INVENTION

According to the present invention, there can be provided a sheet-like structure in which color development is evenly performed, and quantitative accuracy is improved, while maintaining rapidness of a reaction.

### BRIEF DESCRIPTION OF THE DRAWINGS

[Fig. 1] Fig. 1 is a schematic perspective view illustrating a production process of the sheet-like structure of a first embodiment.
[Fig. 2] Fig. 2 is a schematic plan view of a front surface and a back surface of the sheet-like structure of the first embodiment.
[Fig. 3] Fig. 3 is a schematic cross-sectional view of the sheet-like structure of the first embodiment.
[Fig. 4] Fig. 4 is a schematic perspective view illustrating a production process of the sheet-like structure of a second embodiment.
[Fig. 5] Fig. 5 is a schematic cross-sectional view of a front surface and a back surface of the sheet-like structure of the second embodiment.
[Fig. 6] Fig. 6 is a schematic cross-sectional view of the sheet-like structure of the second embodiment.
[Fig. 7] Fig. 7 is a schematic cross-sectional view of the sheet-like structure of a third embodiment.
[Fig. 8] Fig. 8 is a schematic perspective view illustrating a production process of the sheet-like structure of a fourth embodiment.
[Fig. 9] Fig. 9 is a schematic plan view of a front surface and a back surface of the sheet-like structure of the fourth embodiment.
[Fig. 10] Fig. 10 is a schematic cross-sectional view of the sheet-like structure of the fourth embodiment.
[Fig. 11] Fig. 11 is a schematic plan view of a front surface and a back surface of the sheet-like structure of a fifth embodiment.
[Fig. 12] Fig. 12 is a schematic plan view of a front surface and a back surface of the sheet-like structure of a sixth embodiment.
[Fig. 13] Fig. 13 is a schematic plan view of a front surface and a back surface of the sheet-like structure of a seventh embodiment.
[Fig. 14] Fig. 14 is a schematic plan view of a front surface and a back surface of the sheet-like structure of an eighth embodiment.
[Fig. 15] Fig. 15 is a schematic view illustrating Flow Path Pattern 1 in Examples.
[Fig. 16] Fig. 16 is a schematic view illustrating Non-Flow Path Pattern 1 in Examples.
[Fig. 17] Fig. 17 is a schematic view illustrating Flow Path Pattern 2 in Examples.
[Fig. 18] Fig. 18 is a schematic view illustrating Non-Flow Path Pattern 2 in Examples.
[Fig. 19] Fig. 19 is a schematic perspective view illustrating a production process of the sheet-like structure in Comparative Example 1.

### DESCRIPTION OF EMBODIMENTS

### (Sheet-like structure)

The sheet-like structure of the present invention includes a porous structure layer. The porous structure layer includes a flow path that has a porous structure through which a fluid can pass, and a non-flow path that does not allow the fluid to pass through. The flow path has a fluid receiving portion, a flow path portion, and a detection portion. The fluid receiving portion, the flow path portion, and the detection portion are connected in this order so that the fluid can pass through.

A ratio (CFT_{d}/CFT_{f}) of capillary flow time CFT_{d} of the detection portion to capillary flow time CFT_{f} of the flow path portion is 1.2 or greater.

The sheet-like structure is suitably used as an inspection device.

### [Capillary flow time]

The ratio (CFT_{d}/CFT_{f}) is 1.2 or greater. From the viewpoint of evenness of color development, the ratio (CFT_{d}/CFT_{f}) is preferably 1.2 or greater and 5 or less, and more preferably 1.2 or greater and 2.9 or less. From the viewpoint of rapidness of a reaction, the ratio (CFT_{d}/CFT_{f}) is more preferably 1.2 or greater and 2.0 or less.

The capillary flow time (CFT_{f}) of the flow path portion is not particularly limited, and may be appropriately selected depending on the intended purpose. From the viewpoint of rapidness of a reaction, the capillary flow time (CFT_{f}) of the flow path portion is preferably 120 sec/4 cm or less, more preferably 110 sec/4 cm or less, and yet more preferably 100 sec/4 cm or less. In addition, from the viewpoint of ensuring a reaction time, the capillary flow time (CFT_{f}) of the flow path portion is preferably 80 sec/4 cm or greater, more preferably 90 sec/4 cm or greater, and yet more preferably 100 sec/4 cm or greater.

The flow path portion for defining the ratio (CFT_{d}/CFT_{f}) may be at least a part or a whole of the flow path portion in the sheet-like structure.

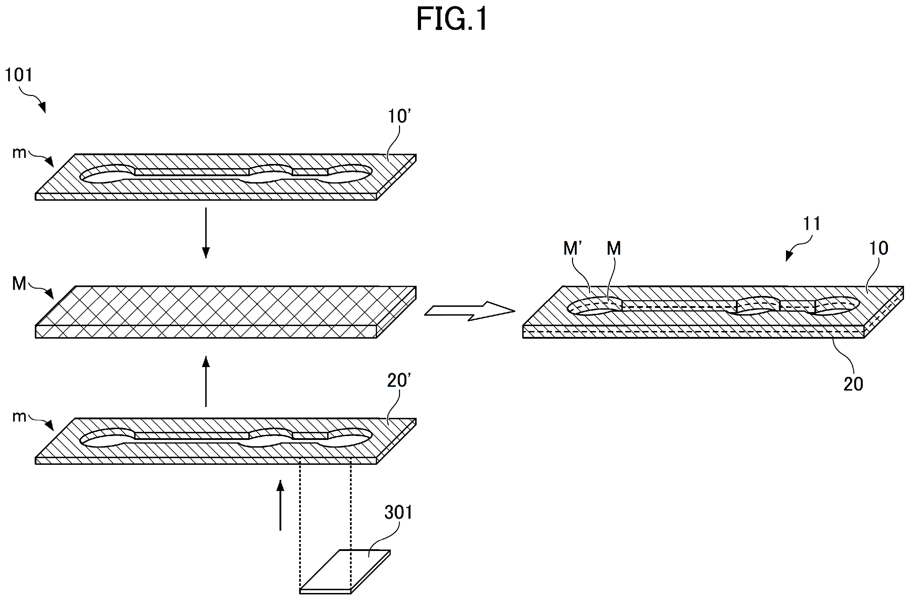

For example, in the case where the sheet-like structure includes multiple flow paths B to D, such as the sheet-like structure of Figs. 1 to 3 below, and the multiple flow paths having different values of CFT_{f}, or in the case where CFT_{f} of the flow path portion is changed stepwise, at least one flow path or at least a part of the flow path may satisfy the defined ratio (CFT_{d}/CFT_{f}), or the entire flow path portion may satisfy the defined ratio (CFT_{d}/CFT_{f}). Among the above, it is preferable that the entire flow path portion satisfies the ratio (CFT_{d}/CFT_{f}).

The capillary flow time (CFT_{d}) of the detection portion is not particularly limited, and may be appropriately selected depending on the intended purpose. From the viewpoint of rapidness of a reaction, the capillary flow time (CFT_{d}) of the detection portion is preferably 300 sec/4 cm or less, more preferably 250 sec/4 cm or less, and yet more preferably 200 sec/4 cm or less. In addition, from the viewpoint of evenness of color development, the capillary flow time (CFT_{d}) of the detection portion is preferably 100 sec/4 cm or greater, more preferably 150 sec/4 cm or greater, and yet more preferably 200 sec/4 cm or greater.

The capillary flow time (CFT) represents the time required for pure water to be soaked up to reach a 4 cm line when one end of a test piece, such as filter paper, is placed upright in the pure water.

The capillary flow time (CFT_{f}) of the flow path portion, the capillary flow time (CFT_{d}) of the detection portion, and the ratio (CFT_{d}/CFT_{f}) of CFT_{d} to CFT_{f} can be evaluated in the following manner.
(1) As a test piece, a strip-shaped sheet-like structure for testing, which has a size of 1 cm × 6 cm, is prepared for each of the flow path portion and the detection portion.
(2) The end portion of the sheet-like structure for testing is vertically immersed in pure water, and the time required for the pure water to be soaked up to reach a 4 cm line of the sheet-like structure for testing is measured.
(3) The measurement is performed using a plurality of test pieces (e.g., n = 3), the average of the measured values is calculated, and the obtained average value is determined as each of capillary flow time (CFT_{f}) of the flow path portion and capillary flow time (CFT_{d}) of the detection portion. Further, a ratio (CFT_{d}/CFT_{f}) of CFT_{d} to CFT_{f} is calculated.

From the viewpoint of the sheet-like structure having the desired CFT_{f}, CFT_{d}, and ratio (CFT_{d}/CFT_{f}), the flow path preferably has the fluid receiving portion and the flow path portion, which communicate with a first surface and a second surface of the porous structure layer, and the detection portion, which communicates with the first surface, but is not exposed at the second surface.

As long as the detection portion communicates with the first surface of the porous structure layer and is not exposed at the second surface of the porous structure, the detection portion may have (1) an embodiment in which a support layer is disposed on the second surface of the detection portion, or (2) an embodiment in which the porous structure at the second surface of the detection portion is impregnated with a hydrophobic material (i.e., an embodiment in which a non-flow path is formed on the second surface side of the detection portion). Any of the above embodiments can be suitably selected.

One surface of the porous structure layer is referred to as a "first surface" or a "front surface," and the opposite surface to the first surface is referred to as a "second surface" or a "back surface." The above terms are merely used for facilitating understanding of the invention with reference to drawings, but the meaning of the above terms does not limit the technical scope of the present invention.

From the viewpoint of desired CFT_{f}, the flow path portion preferably has an embodiment in which the flow path portion communicates with the first surface and the second surface of the porous structure layer, and the embodiment may be (1) an embodiment in which a support layer is not provided, and the first surface and the second surface of the porous structure layer are exposed or (2) an embodiment in which a support layer is disposed on the second surface of the flow path portion (i.e., an embodiment in which the second surface of the flow path portion is not exposed). Any of the above embodiments can be suitably selected.

Similarly to the flow path portion, the fluid receiving portion preferably has an embodiment in which the fluid receiving portion communicates with the first surface and the second surface of the porous structure layer, and the embodiment may be (1) an embodiment in which a support layer is not provided, and the first surface and the second surface of the porous structure layer are exposed or (2) an embodiment in which a support layer is disposed on the second surface of the fluid receiving portion (i.e., an embodiment in which the second surface of the fluid receiving portion is not exposed). Any of the above embodiments can be suitably selected.

The support layer is preferably impermeable to water.

The present invention will be described in detail based on several embodiments hereinafter, but the present invention is not limited to the following descriptions of the embodiments in any way.

### <First embodiment>

The sheet-like structure 11 of a first embodiment will be specifically described with reference to Figs. 1 to 3.

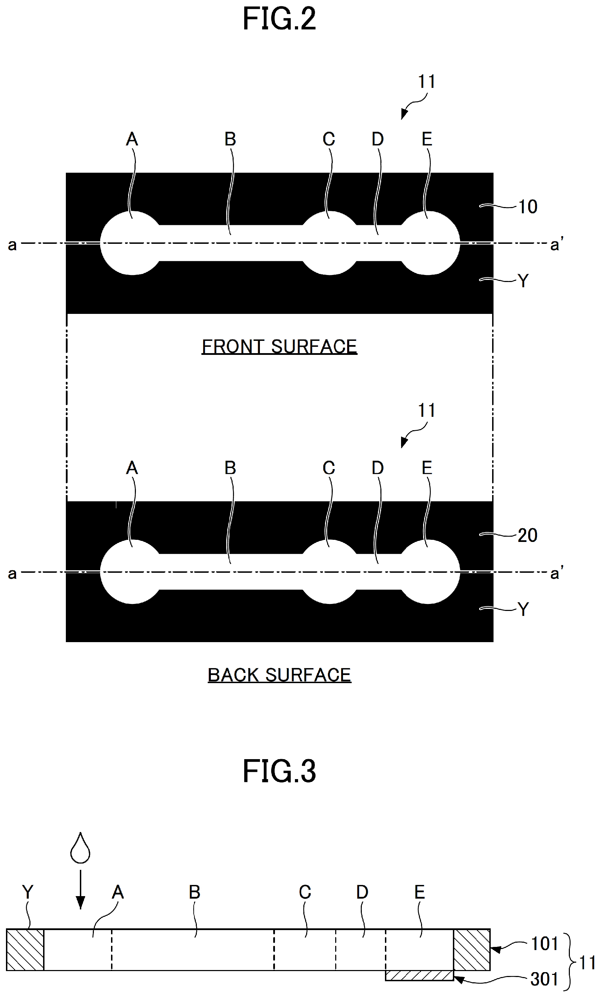

Fig. 1 is a schematic perspective view illustrating a production process of the sheet-like structure of the first embodiment. Fig. 2 is a schematic plan view of a front surface and a back surface of the sheet-like structure of the first embodiment. Fig. 3 is a schematic cross-sectional view of the sheet-like structure of the first embodiment.

The sheet-like structure 11 of the first embodiment illustrated in Figs. 1 to 3 includes a porous structure layer 101 including a flow path X having a porous structure trough which a fluid can pass, and a non-flow path Y that does not allow the fluid to pass through. The flow path X has a fluid receiving portion A, flow path portions B, C, and D, and a detection portion E. The fluid receiving portion A, the flow path portions B, C, and D, and the detection portion E are connected in this order so that the fluid can pass through. The fluid receiving portion A and the flow path portions B, C, and D communicate with a front surface and a back surface of the porous structure layer 101. The sheet-like structure 11 further includes a support layer 301 to cover the back surface of the detection portion E so that the back surface side of the detection portion E is not exposed.

Since the sheet-like structure 11 has such a configuration, the ratio (CFT_{d}/CFT_{f}) can be 1.2 or greater, evenness of color development is achieved, and quantitative accuracy can be improved, while maintaining the flow rate of the fluid to maintain rapidness of a reaction.

A method for producing the sheet-like structure 11 is not particularly limited, and may be appropriately selected depending on the intended purpose. For example, the sheet-like structure 11 can be suitably produced by the production process illustrated in Fig. 1. As schematically illustrated in Fig. 1, both surfaces of a material M having a porous structure are impregnated with a hydrophobic material m having a pattern of a non-flow path Y from which a flow path pattern of a flow path X is absent, thereby obtaining a porous structure layer 101 in which the non-flow path Y is formed of the material M' that is the material M impregnated with the hydrophobic material m. The flow paths A to E are formed of the material M having the porous structure, and the non-flow path Y is formed of the material M', which is the material M impregnated with the hydrophobic material.

Subsequently, a support layer 301 is disposed on the back surface of the porous structure layer 101 to cover the back surface of the detection portion E, thereby producing the sheet-like structure 11 of the first embodiment.

### -Porous structure layer -

The porous structure layer 101 of the sheet-like structure 11 is provided with the flow path X having a porous structure through which a fluid can pass due to a capillary action or the like, and the non-flow path Y provided in the region other than the flow path X. The flow path X has a fluid receiving portion A, flow path portions B, C, and D, and a detection portion E, and the fluid receiving portion A, the flow path portions B, C, and D, and the detection portion E are connected in this order so that the fluid can pass through.

The "fluid" is not particularly limited as long as the fluid is a fluid that can be passed through the flow path composed of the porous structure by a capillary action or the like, and may be appropriately selected according to the intended purpose. In the case where the sheet-like structure of the present invention is applied for an inspection device, examples of the fluid include a solution including a test sample that is suspected to include a detection target substance, a solution including a test sample and a reaction reagent, and the like.

The viscosity of the fluid is not particularly limited as long as the viscosity of the fluid is a viscosity at which the fluid can be passed through the flow path composed of the porous structure, and may be appropriately adjusted according to the intended purpose.

The fluid receiving portion A is a portion on which the fluid is dropped. In addition, the detection portion E is a portion with which the presence or absence of a detection target substance, such as an antigen, in the fluid dropped on the fluid receiving portion A is confirmed by the presence or absence of coloration. The reaction reagent may be included in the fluid, and may be arranged on the flow path of the sheet-like structure. For example, the reaction reagent can be arranged on the flow path portion C of the sheet-like structure 11.

In Figs. 1 to 3, the flow path portions having different flow path widths or shapes are provided as the flow path portions B to D, but an integrated flow path portion having a constant flow path width may be provided.

The "porous structure" is a structure including multiple communicating pores, and may be generally referred to as a co-continuous structure or a monolithic structure. In the porous structure, continuously connected pores are threedimensionally spread, thereby allowing a fluid to penetrate (i.e., a capillary action).

A cross-sectional shape of each pore in the porous structure can be appropriately set considering physical properties of a fluid, such as viscosity or the like. Examples of the cross-sectional shape of each pore include substantially circular shapes, substantially elliptical shapes, substantially polygonal shape, and the like. The size of the pores in the porous structure is not particularly limited, and may be appropriately selected according to the intended purpose. The cross-sectional shapes and size of the pores can be determined, for example, from a cross-sectional photograph captured by a scanning electron microscope (SEM) or the like.

The porosity of the porous structure can be appropriately set considering physical properties of a fluid, such as viscosity, or the like. A method of measuring the porosity is not particularly limited. Examples of the method include a method in which a porous structure is filled with unsaturated fatty acid (commercially available butter), osmium staining is performed, followed by cutting out an inner cross-sectional structure by a focused ion beam (FIB), and a porosity is measured using a scanning electron microscope (SEM), and the like.

The distribution of the pores within the porous structure can be appropriately set considering physical properties of a fluid, such as viscosity or the like, as long as a fluid can pass through the porous structure. The pores are preferably uniformly distributed within the flow path region.

The shape of the flow path X in a plan view is not particularly limited as long as a fluid can be passed through the flow path, and may be appropriately selected according to the intended purpose. Examples of the shape include circular shapes, elliptical shapes, square shapes, rectangular shapes, and the like.

The diameter (or major axis) of the flow path X is not particularly limited, and may be appropriately selected according to the intended purpose. For example, the diameter can be set to 3 mm or greater and 10 mm or less.

The flow path width of each of the flow path portions B to D is not particularly limited, and may be appropriately selected according to the intended purpose. For example, the flow path width can be set to 1 mm or greater and 5 mm or less.

The material M of the flow path X is not particularly limited as long as the material M has a porous structure through which a fluid can pass, and may be appropriately selected according to the intended purpose. Examples of the material M include paper, such as filter paper, non-woven fabrics, nitrocellulose, polypropylene, and the like.

Among the above materials, filter paper is more preferred in view of simplicity and low cost.

The non-flow path Y is a region of the porous structure layer excluding the flow path X, specifically, a region where passing of a fluid does not occur.

The material M' of the non-flow path Y is not particularly limited as long as passing of a fluid does not occur, and may be appropriately selected according to the intended purpose. For example, the material M' can be obtained by impregnating the material M with a hydrophobic material m.

The hydrophobic material is preferably a material having a melting point of 90°C or lower, from the viewpoint of easy production of the porous structure layer. Examples of the material M' include wax, compositions including wax, and the like.

In addition to the hydrophobic material, a viscosity adjusting component, such as a resin or the like, dispersion aids, fillers and the like can be appropriately added to the composition.

In the case where the material M is impregnated with the hydrophobic material, the hydrophobic material is preferably melted by heating in advance. The heating temperature can be appropriately set considering a melting point of the hydrophobic material or a melting point of a viscosity adjusting component.

The viscosity of the hydrophobic material when melted can be appropriately set so that the porous structure layer can be impregnated with the hydrophobic material as desired, considering an average thickness, basis weight (density), or the like of the porous structure layer.

In the case where the material M of the flow path is impregnated with the hydrophobic material, an impregnation ratio of the hydrophobic material relative to the material M is preferably within the range of 14% or greater and 32% or less.

By producing the porous structure layer to have the impregnation ratio of 14% or greater, a wall surface (an interface between the material M and the material M') of the flow path becomes a sufficiently even surface so that passing of a fluid, for example, from the flow path to another flow path, can be made smoother. By producing the porous structure layer to have the impregnation ratio of 32% or less, a problem, such as blockage, is sufficiently avoided when the material M is impregnated with the hydrophobic material. Thus, a porous structure layer having a desired flow path structure can be more assuredly obtained.

The "impregnation ratio" refers to a fraction of the material M' when the state in which the material M is saturated with the hydrophobic material in the region of the porous structure layer having the material M' across the entire thickness direction is determined as an impregnation ratio of 100% (the maximum impregnation amount).

The state in which the material M is saturated with the hydrophobic material (the maximum impregnation amount) is appropriately determined according to a combination of the material M and the hydrophobic material. For example, the material M is impregnated with the hydrophobic material that is heated (e.g., heated at 120°C) to have a sufficiently low viscosity, and the resultant material M is left to stand for a sufficient time (e.g., 3 minutes) while holding the temperature, thereby obtaining a material M'. The obtained material M' is determined as having the impregnation ratio of 100%. The adjustment of the impregnation ratio can be performed, for example, by adjusting an amount of the hydrophobic material to be impregnated (e.g., a thickness of a composition including the hydrophobic material or the like).

A measuring method for the impregnation ratio of the hydrophobic material relative to the material M is not particularly limited, and may be appropriately selected according to the intended purpose. Examples of the measuring method include the following method.

### [Measuring method for impregnation ratio]

Filter paper is cut into a size of 5 cm × 2 cm, and dried at 120°C for 3 minutes. Then, a dry mass M₀ (g) of the filter paper is measured. Subsequently, the filter paper is impregnated with a hydrophobic material, and is left to stand at 120°C for 3 minutes. The impregnated filter paper is held between the same type of filter paper and glass slides, and is left to stand at 120°C for 1 minute with a load of 100 gf to remove the excess hydrophobic material. Thereafter, a mass M₁ (g) of the filter paper is measured, and the maximum impregnation amount Pₘₐₓ (g/m²) per unit area is calculated according to the following equation (1). ${P}_{max} \left({g / m}^{2}\right) = \left({M}_{1}-{M}_{0}\right) \times 1 , 000$ Equation (1)

Subsequently, the impregnation amount P (g/m²) of the hydrophobic material in the material M impregnated with the hydrophobic material serving as the measurement target is calculated, and the impregnation ratio is calculated according to the following equation (2). $Impregnation ratio \left(%\right) = \left({P / P}_{max}\right) \times 100$ Equation (2)

The viscosity of the composition including the hydrophobic material is not particularly limited, and may be appropriately selected depending on the intended purpose. For example, for sufficiently avoiding a problem, such as blockage during the impregnation of the material M, the viscosity of the composition at 140°C and at the shearing speed of 3,000 s⁻¹ is preferably 100 mPa·s or lower, more preferably 50 mPa·s or lower, and yet more preferably 30 mPa·s or lower.

Although the measurement of the viscosity is not particularly limited, the viscosity can be measured, for example, by a rheometer (e.g., product name: AR-G2 rheometer, produced by TA Instruments Inc.).

The material M' is preferably colored so that passing of a fluid can be easily visually recognized, but may be white or transparent, or may not be colored. The coloring of the material M' can be achieved, for example, by impregnating the material M with a colorant in addition to the hydrophobic material.

Examples of the colorant include pigments, such as carbon black (a black pigment) and the like. The colorant is preferably hydrophobic. Moreover, as the colorant, a colorant that does not adversely affect a reagent used for an inspection or the like is preferably selected.

The shape of the porous structure layer 101 in a plan view is not particularly limited, and may be appropriately selected depending on the intended purpose. Examples of the shape of the porous structure layer 101 include rectangular shapes, substantially circular shapes, substantially elliptical shapes, substantially rectangular shapes, and the like.

### -Support layer-

The sheet-like structure 11 includes a support layer 301.

The support layer 301 is disposed to cover the second surface of the detection portion E of the sheet-like structure 11. Since the second surface of the detection portion E is not exposed, the capillary flow time CFT_{d} of the detection portion becomes relatively long with respect to the capillary flow time CFT_{f} of the flow path portions B to D so that a desired ratio (CFT_{d}/CFT_{f}) is obtained. Further, the support layer 301 has a function of reinforcing the physical strength of the sheet-like structure 11 itself.

The material of the support layer 301 is not particularly limited, and may be appropriately selected according to the intended purpose. The material of the support layer 301 is preferably impermeable to water. Examples of the material include polypropylene and the like.

The size, structure, and shape of the support layer 301 are not particularly limited as long as the support layer can cover the flow path of the porous structure layer, and may be appropriately selected according to the intended purpose.

As the support layer 301, a commercially available product can be used. Examples of the commercially available product include a product by the name of 660-PF (manufactured by NICHIBAN Co., Ltd.) and the like.

The average thickness of the sheet-like structure 11 is not particularly limited, and may be appropriately selected according to the intended purpose. For example, the average thickness of the sheet-like structure 11 can be 100 µm or greater and 300 µm or less.

The average thickness of the sheet-like structure 11 can be measured, for example, by a thickness gauge (e.g., , ID-C112BS, manufactured by Mitutoyo Corporation).

The size of the sheet-like structure 11 of the first embodiment is not particularly limited, and may be appropriately selected according to the intended purpose.

### <Second embodiment>

Although the first embodiment is an embodiment in which the unexposed second surface of the detection portion E is covered with the support layer 301, it may be an embodiment in which the porous structure at the second surface of the detection portion E may be impregnated with a hydrophobic material, in other words, an embodiment in which the sheet-like structure has a non-flow path on the second surface side of the detection portion E, as in the second to fourth embodiments, which will be described hereinafter.

Hereinafter, the configuration different from the first embodiment will be mainly described. The features common to those in the first embodiment may be appropriately selected from the features described in the first embodiment.

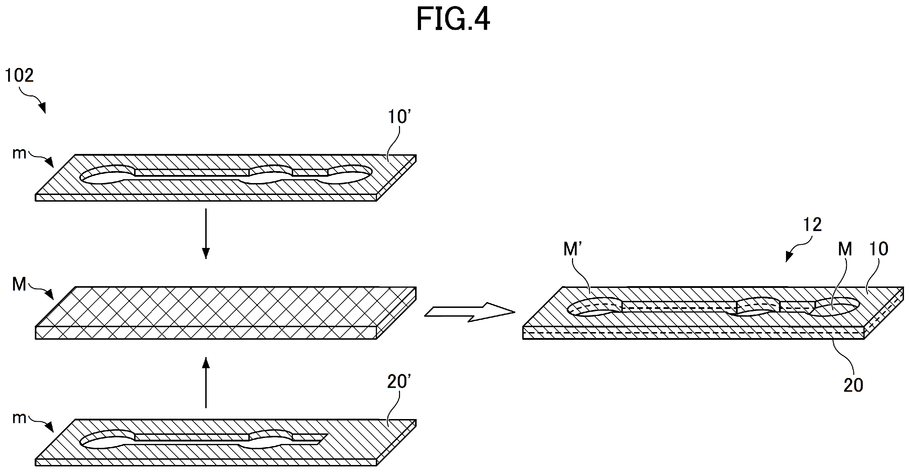

The sheet-like structure 12 of the second embodiment will be specifically described with reference to Figs. 4 to 6.

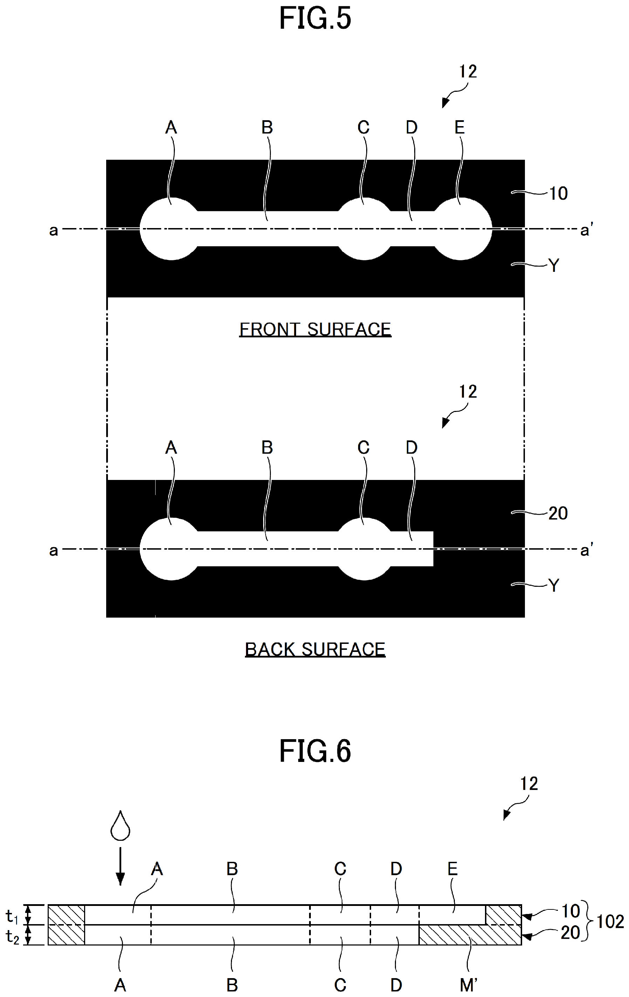

Fig. 4 is a schematic perspective view illustrating a production process of the sheet-like structure of the second embodiment. Fig. 5 is a schematic plan view of a front surface and a back surface of the sheet-like structure of the second embodiment. Fig. 6 is a schematic cross-sectional view of the sheet-like structure of the second embodiment.

The sheet-like structure 12 of the second embodiment illustrated in Figs. 4 to 6 includes a porous structure layer 102 including a flow path X having a porous structure through which a fluid can pass, and a non-flow path Y that does not allow the fluid to pass through. The flow path X has a fluid receiving portion A, flow path portions B, C, and D, and a detection portion E, and the fluid receiving portion A, the flow path portions B, C, and D, and the detection portion E are connected in this order so that the fluid can pass through. The fluid receiving portion A and the flow path portions B, C, and D communicate with the front surface and the back surface of the porous structure layer 102. The sheet-like structure 12 further includes a non-flow path on the second surface side of the detection portion E, where the non-flow path is formed by impregnating the porous structure 20 at the back surface of the detection portion E with a hydrophobic material, so that the back surface side of the detection portion E is not exposed.

Since the sheet-like structure 12 has such a configuration, the ratio (CFT_{d}/CFT_{f}) can be 1.2 or greater, evenness of color development is achieved, and quantitative accuracy can be improved, while maintaining the flow rate of the fluid to maintain rapidness of a reaction.

A method for producing the sheet-like structure 12 is not particularly limited, and may be appropriately selected depending on the intended purpose. For example, the sheet-like structure 12 can be suitably produced by the production process illustrated in Fig. 4. As schematically illustrated in Fig. 4, both sides of the material M having the porous structure are impregnated with a hydrophobic material m having a pattern of a non-flow path Y from which a flow path pattern of the flow path X is absent, thereby obtaining a porous structure layer 102 with the non-flow path Y, which is formed of the material M' in which the material M is impregnated with the hydrophobic material m.

The shape of the hydrophobic material m for the front surface and the shape of the hydrophobic material m for the back surface are different from each other. The hydrophobic material m for the front surface has a non-flow path pattern from which the flow path pattern of the flow paths A to E is absent, whereas the hydrophobic material m for the back surface has a non-flow path pattern from which only the flow path pattern of the flow paths A to D is absent. Therefore, the back surface of the detection portion E of the porous structure 20 is impregnated with the hydrophobic material, thereby producing the sheet-like structure 12 of the second embodiment, which has the non-flow path at the second surface side of the detection portion E.

Further, in Fig. 6, an edge region obtained when the porous structure layer 102 is impregnated with the hydrophobic material from the front surface of the porous structure layer 102 is drawn by a dotted line as a virtual line. The virtual line also indicates the edge region obtained when the porous structure layer 102 is impregnated with the hydrophobic material from the back surface of the porous structure layer 102, and is also a boundary between both edge regions. With the virtual line as a boundary, the front surface side of the porous structure layer 102 is referred to as a porous structure 10, and the back surface side of the porous structure layer 102 is referred to as a porous structure 20. The virtual line is similarly drawn in other drawings.

In the sheet-like structure 12, a ratio (t2/t1) of the average thickness (t1) of the porous structure 10 to the average thickness (t2) of the porous structure 20 is approximately 1. The sheet-like structure 12 having such a configuration can be suitably produced by making the average thickness of the hydrophobic material m for the front surface and the average thickness of the hydrophobic material m for the back surface substantially the same in the production process illustrated in Fig. 4.

### <Third embodiment>

Although the second embodiment is an embodiment in which the ratio (t2/t1) of the average thickness (t2) of the porous structure 20 to the average thickness (t1) of the porous structure 10 is approximately 1, it may be an embodiment in which the ratio (t2/t1) may be changed as in the third embodiment that will be described hereinafter.

Hereinafter, the configuration different from the second embodiment will be mainly described. The features common to those in the first and second embodiments may be appropriately selected from the features described in the first and second embodiments.

The sheet-like structure 13 of the third embodiment will be specifically described with reference to Figs. 4, 5, and 7.

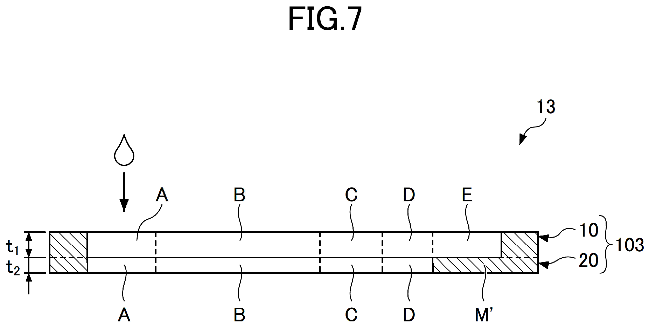

The sheet-like structure 13 of the third embodiment illustrated in Fig. 7 is different from the sheet-like structure 12 of the second embodiment in that the ratio (t2/t1) of the average thickness (t2) of the porous structure 20 to the average thickness (t1) of the porous structure 10 is less than 1. The schematic plan view of the front surface and the back surface of the sheet-like structure of the third embodiment is the same as that of the second embodiment illustrated in Fig. 5.

A method for producing the sheet-like structure 13 is not particularly limited, and may be appropriately selected depending on the intended purpose. For example, the sheet-like structure 13 can be suitably produced by adjusting a ratio of the average thickness of the hydrophobic material m for the back surface and the average thickness of the hydrophobic material m for the front surface in the production process in the second embodiment illustrated in Fig. 4.

The ratio (t2/t1) is not particularly limited, and may be appropriately selected depending on the intended purpose, but is preferably 0.56 or greater and 2.2 or less.

By producing a sheet-like structure so that the ratio (t2/t1) is 0.56 or greater and 2.2 or less, a problem, such as blockage, when the material M is impregnated with the hydrophobic material m, is sufficiently avoided, and a flow rate, speed stability, or both the flow rate and speed stability of a fluid, for example, from the flow path A to the flow path D can be effectively increased.

From the viewpoint of improvement in evenness of color development through an increase in the ratio (CFT_{d}/CFT_{f}) in the second embodiment and the third embodiment, the ratio (t2/t1) is preferably 0.6 or greater, more preferably 0.8 or greater, and yet more preferably 1.0 or greater. The ratio (t2/t1) is not particularly limited, and may be 3.0 or less.

### <Fourth embodiment>

As demonstrated with the sheet-like structure 14 of the fourth embodiment, it may be an embodiment in which the sheet-like structure 12 of the second embodiment further includes a support layer 304 on the entire back surface of the porous structure layer 104.

Hereinafter, the configuration different from the second embodiment will be mainly described. The features common to those in the first to third embodiments may be appropriately selected from the features described in the first to third embodiments.

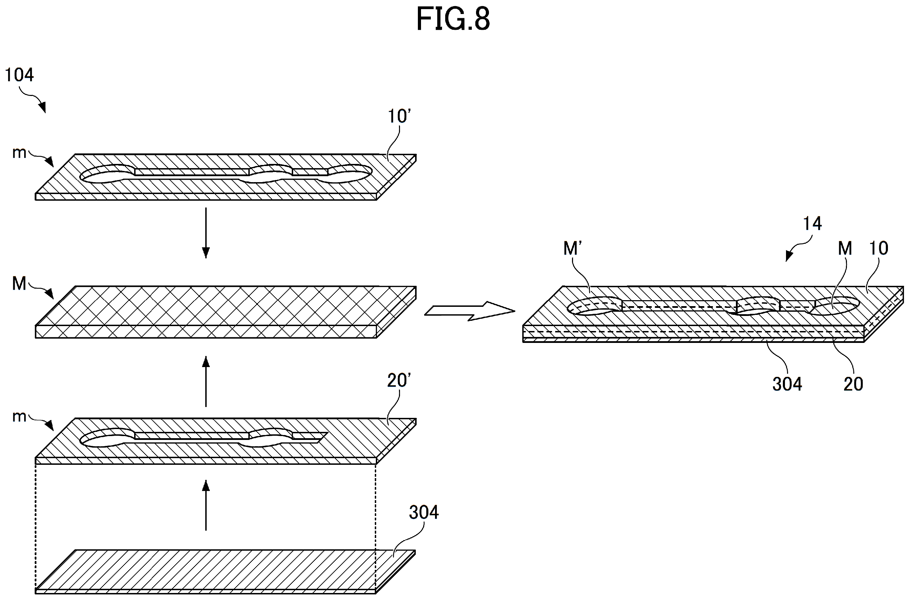

The sheet-like structure 14 of the fourth embodiment will be specifically described with reference to Figs. 8 to 10.

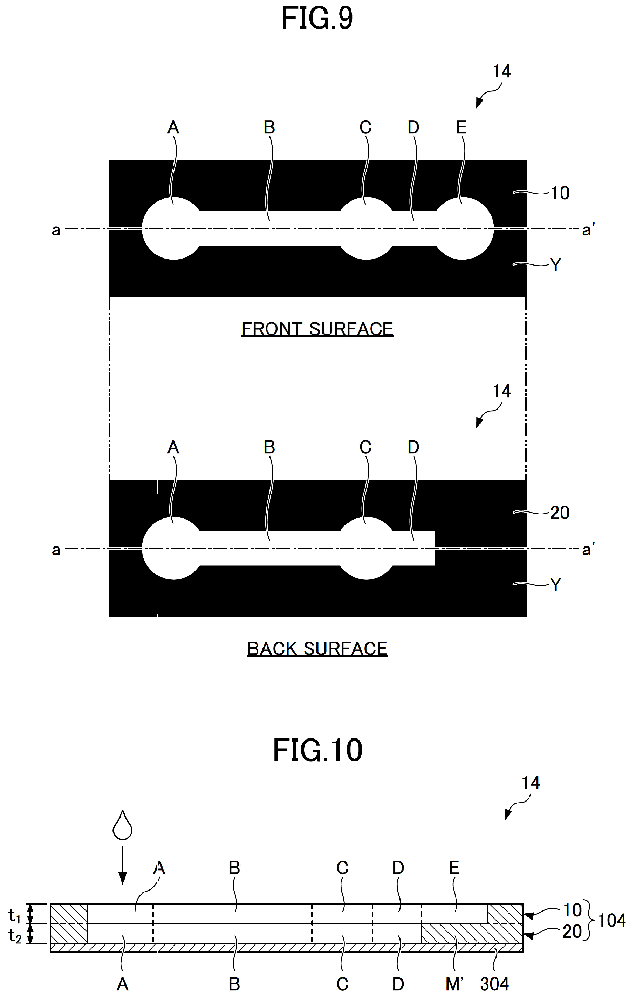

Fig. 8 is a schematic perspective view illustrating a production process of the sheet-like structure of the fourth embodiment. Fig. 9 is a schematic plan view of a front surface and a back surface of the sheet-like structure of the fourth embodiment. Fig. 10 is a schematic cross-sectional view of the sheet-like structure of the fourth embodiment.

The sheet-like structure 14 of the fourth embodiment illustrated in Figs. 8 to 10 includes a porous structure layer 104 including a flow path X having a porous structure through which a fluid can pass, and a non-flow path Y that does not allow the fluid to pass through. The flow path X includes a fluid receiving portion A, flow path portions B, C, and D, and a detection portion E, and the fluid receiving portion A, the flow path portions B, C, and D, and the detection portion E are connected in this order so that the fluid can pass through. The fluid receiving portion A and the flow path portions B, C, and D communicate with the front surface and the back surface of the porous structure layer 104. The sheet-like structure 14 includes a non-flow path on the second surface side of the detection portion E, where the non-flow path is formed by impregnating the porous structure 20 at the back surface of the detection portion E with a hydrophobic material. The sheet-like structure 14 further includes a support layer 304 on the entire back surface of the porous structure layer 104 so that the back surface side of the fluid receiving portion A and the flow path portions B, C, and D is not exposed.

Since the sheet-like structure 14 has such a configuration, rapidness of a reaction can be maintained while ensuring a reaction time of a fluid in the fluid receiving portion A and the flow path portions B, C, and D, the ratio (CFT_{d}/CFT_{f}) can be 1.2 or greater, evenness of color development is achieved, and quantitative accuracy can be improved.

In addition, the physical strength of the sheet-like structure 14 itself can be reinforced by the support layer 304. Further, the fluid passing through the flow path can be prevented from being contaminated. For example, in the case where the fluid is a test sample and a reagent (e.g., an antigen and an antibody, or the like), it is possible to prevent reaction inhibition or the like due to contamination, which is preferable.

A method for producing the sheet-like structure 14 is not particularly limited, and may be appropriately selected depending on the intended purpose. For example, the sheet-like structure 14 can be suitably produced by the production process illustrated in Fig. 8. As schematically illustrated in Fig. 8, both surfaces of the material M having the porous structure are impregnated with the hydrophobic material m having the non-flow path Y for the front surface and the hydrophobic material m having the non-flow path Y for the back surface, which are different from each other, respectively, and therefore, the back surface of the detection portion E of the porous structure 20 is impregnated with the hydrophobic material, thereby obtaining a porous structure layer 104 having a non-flow path on the second surface side of the detection portion E. The flow paths A to E are composed of the material M having the porous structure, and the non-flow path Y is composed of the material M' in which the material M is impregnated with the hydrophobic material.

Subsequently, a support layer 304 is disposed on the back surface of the porous structure layer 104 to cover the back surface of the fluid receiving portion A and the flow path portions B, C, and D, thereby producing the sheet-like structure 14 of the fourth embodiment.

Although the support layer 304 is provided on the entire back surface of the porous structure layer 104 in the fourth embodiment, the sheet-like structure is not limited to such an embodiment.

The region in which the support layer 304 is disposed is not particularly limited, and may be appropriately selected depending on the intended purpose. The support layer 304 may be disposed only on the back surface of the fluid receiving portion A, only on the back surface of the flow path portions B, C, and D, or on the entire back surface or any part of the back surface of the flow path.

An additional support layer 304' may be disposed on the front surface of the porous structure layer 104 as long as the ratio (CFT_{d}/CFT_{f}) of 1.2 or greater is satisfied. The region in which the support layer 304' is disposed may be the front surface of the non-flow path to expose the flow paths A to D, or may include the front surface of any of the flow path portions B, C, and D.

### <Fifth embodiment to eighth embodiment>

A plurality of flow paths may be provided as in the fifth to eighth embodiments described below.

Hereinafter, the fifth to eighth embodiments in which a plurality of flow paths are provided based on the second embodiment will be described, but a plurality of flow paths may be provided instead of one flow path in each of the first embodiment, the third embodiment, and the fourth embodiment. The features common to those in the first to fourth embodiments may be appropriately selected from the features described above.

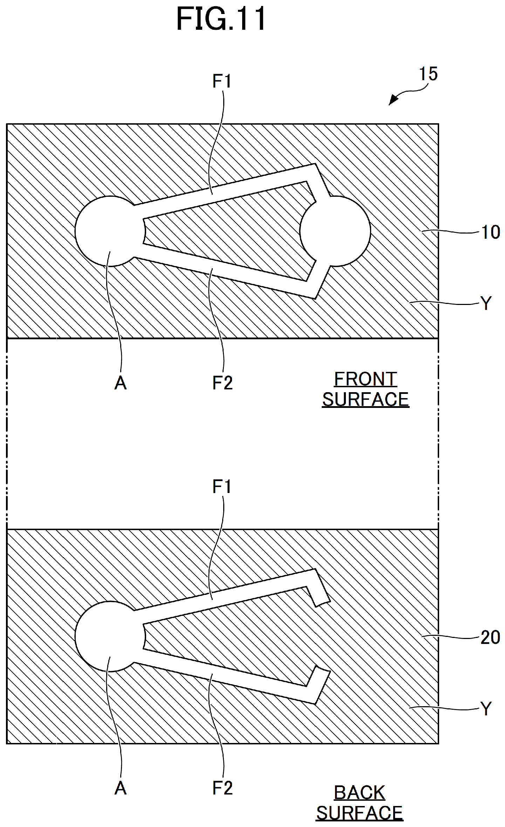

Fig. 11 is a schematic plan view of a front surface and a back surface of the sheet-like structure of the fifth embodiment.

As illustrated in Fig. 11, the sheet-like structure 15 of the fifth embodiment is the same as that of the second embodiment illustrated in Figs. 4 to 6, except that the sheet-like structure 15 has a plurality of the flow path portions F (two portions, F1 and F2 in Fig. 11). In Fig. 11, the flow path portions F each having a substantially constant flow path width are provided, but flow path portions B to D having different flow path widths or shapes may be provided as in the first to fourth embodiments, instead of the flow path portions F.

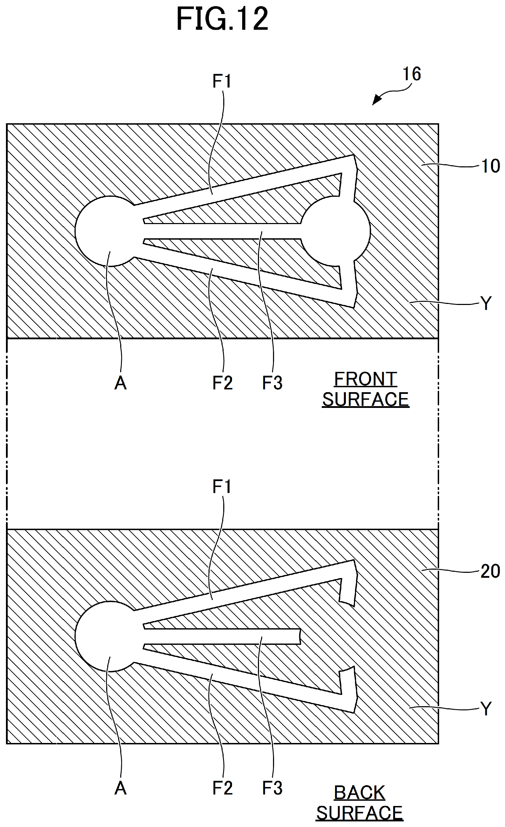

Fig. 12 is a schematic plan view of a front surface and a back surface of the sheet-like structure of the sixth embodiment.

As illustrated in Fig. 12, the sheet-like structure 16 of the sixth embodiment is substantially the same as that of Fig. 11, except that the sheet-like structure 16 has a configuration having three flow path portions F (including F3 in addition to F1 and F2). In the sheet-like structure 16 illustrated in Fig. 12, the three flow path portions F are connected to the detection portion E in a manner such that the flow path portions F face one another.

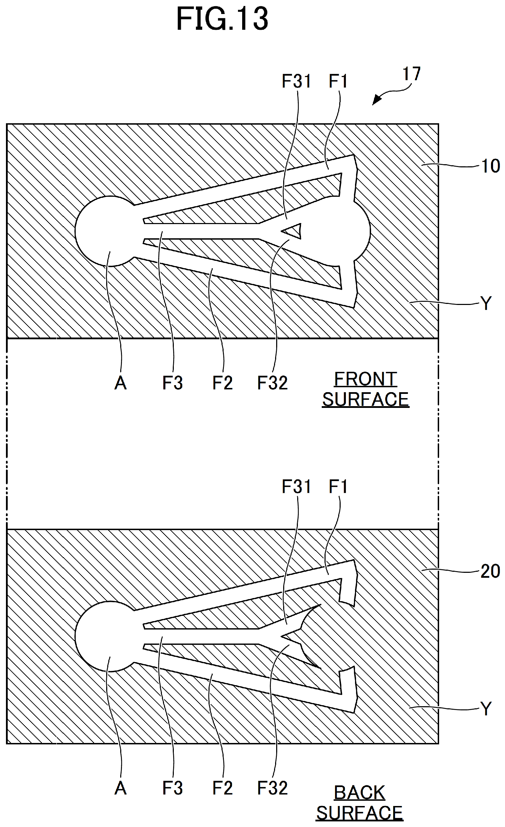

Fig. 13 is a schematic plan view of a front surface and a back surface of the sheet-like structure of the seventh embodiment.

As illustrated in Fig. 13, the sheet-like structure 17 of the seventh embodiment is substantially the same as that of Fig. 12, except that the sheet-like structure 17 has a configuration in which the flow path portion F3 is branched into two portions (F31 and F32), and the branched flow paths F31 and F32 are connected to the detection portion E.

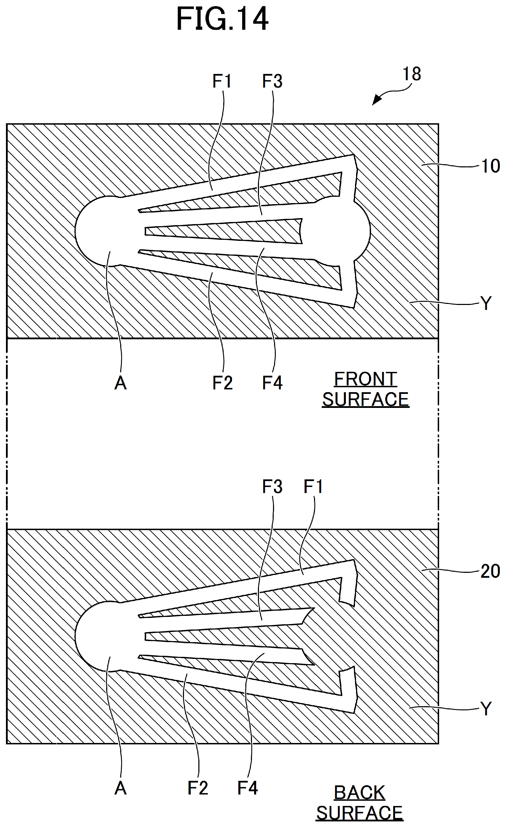

Fig. 14 is a schematic plan view of a front surface and a back surface of the sheet-like structure of the eighth embodiment.

As illustrated in Fig. 14, the sheet-like structure 18 of the eighth embodiment is substantially the same as that of Fig. 11, except that the sheet-like structure 18 has a configuration including two flow path portions F (F3 and F4) in addition to the flow path portion F1 and the flow path portion F2. In the sheet-like structure 18 illustrated in Fig. 14, the four flow path portions F are connected to the detection portion E in a manner such that the four flow path portions F face one another.

In the sheet-like structure as illustrated in Figs. 11 to 14, the number of the flow path portions F is preferably 4 or less, more preferably 3 or less, and yet more preferably 2, from the viewpoint of inhibition of the increase in the fluid amount. In addition, the number of the connection points between the flow path portions F and the detection portion E is preferably 4 or less, more preferably 3 or less, and yet more preferably 2.

Among a plurality of the flow path portions F, at least two flow path portions F are preferably connected to the detection portion E in a manner such that the at least two flow path portions F face each other. In addition, among a plurality of the flow path portions F, at least two flow path portions F preferably have substantially the same shape.

The sheet-like structure can be produced, for example, by forming a flow path and a non-flow path for a front surface on a front surface of a sheet-like material to produce a first porous structure layer, forming a flow path and a non-flow path for a back surface on a back surface of a sheet-like material to produce a second porous structure layer, and laminating the first porous structure layer and the second porous structure layer.

Alternatively, the sheet-like structure can be produced by forming a flow path and a non-flow path for a front surface on a part of a single sheet-like material to produce a first porous structure layer, forming a flow path and a non-flow path for a back surface on another part of the single sheet-like material to produce a second porous structure layer, and folding the sheet-like material while adjusting the positions of the first porous structure layer and the second porous structure layer.

The sheet-like structure of the present invention is preferably produced by forming a first porous structure layer on one side (a front surface side) of a single sheet-like material, and forming a second porous structure layer on the other side (a back surface side) of the single sheet-like material. The above sheet-like structure in which the first porous structure layer and the second porous structure layer are respectively formed on corresponding sides of the single sheet-like material has various advantages, such as (1) the labor and cost for laminating (or folding) can be avoided, (2) passing of a fluid between the first porous structure layer and the second porous structure layer by a capillary action can be ensured, (3) disposal is easily performed because a tool for retaining the laminate (fold) of the sheet-like materials or the like is not necessary, and the like.

As a specific production method of the sheet-like structure, for example, the sheet-like structure can be produced by the following method.

### [Method for producing sheet-like structure]

First, a hydrophobic material, a colorant, and a resin are blended. The resultant mixture is melted and mixed, for example, at 100°C or higher and 140°C or lower, thereby preparing a wax ink. The prepared wax ink is applied onto a substrate, such as a polyethylene terephthalate film, thereby producing an ink ribbon. Next, a specific flow path pattern is printed on wood-free paper by a thermal transfer printer (e.g., device name: L'esprit R412v-ex, manufactured by SATO HOLDINGS CORPORATION), thereby forming a non-flow path pattern that is a pattern corresponding to a printing portion of the ink ribbon from which the flow path pattern is absent. After fixing pieces of the ink ribbon having the non-flow path pattern on corresponding front and back sides of filter paper, the resultant stack is passed through a laminator (e.g., device name: GL535ML, manufactured by GBG) set at a predetermined temperature and line speed to transfer the wax ink to filter paper and allow the wax ink to transfer and penetrate into the filter paper to form a three-dimensional flow path, thereby producing a sheet-like structure.

The filter paper is not particularly limited, and may be appropriately set according to the intended purpose. For example, filter paper having an average thickness of 220 µm, a basis weight of 85 g/m³, and capillary flow time (CFT) of 95.1 [sec/4 cm] can be used.

The "predetermined temperature and line speed" are not particularly limited as long as the predetermined temperature and line speed are conditions under which the wax ink can penetrate into and be transferred to the filter paper. For transferring, the conditions can be set at 85°C and at the line speed of 10 mm/sec. For penetration, the conditions can be set at 85°C and at the line speed of 5 mm/sec.

From the viewpoint of preventing a passing failure and adsorption of a fluid (a test sample, such as an antigen, and a reagent, such as an antibody), a blocking agent may be applied to the fluid receiving portion A and the detection portion E in advance. Examples of the blocking agent include an albumin aqueous solution and the like. The blocking agent is preferably appropriately selected depending on the type of the fluid, or the physical properties of the fluid, such as viscosity or the like.

### (Inspection device)

The sheet-like structure of the present invention can be suitably used as an inspection device. Examples of the inspection device include pregnancy test kits, inspection devices using a measuring method called an immunochromatography method in which the principle of sandwich ELISA and the principle of chromatography are combined, and the like.

### Examples

Next, the present invention will be more specifically described through Examples and Comparative Examples, but the present invention is not limited to Examples below.

### <Preparation of ink ribbon>

The following materials were blended, and the resultant mixture was melted and mixed at 100°C to prepare a wax ink.
·Paraffin wax as a hydrophobic material (product name: Paraffin Wax-135, manufactured by NIPPON SEIRO CO., LTD.): 72.0 parts by mass
·Synthetic wax as a hydrophobic material (product name: DIACARNA (registered trademark) 30, manufactured by Mitsubishi Chemical Corporation): 18.0 parts by mass
·Carbon black as a colorant (product name: MA-100, manufactured by Mitsubishi Chemical Corporation): 1.8 parts by mass
·Resin (product name: Ultrathene (registered trademark) 722, manufactured by Tosoh Corporation): 11.25 parts by mass

The viscosity of the obtained wax ink under the conditions of 140°C and the shearing speed of 3,000 s⁻¹ was 23 mPa·s. The viscosity was measured by Rheometer AR-G2 (produced by TA Instruments Inc.).

The obtained wax ink was applied onto a polyethylene terephthalate film having the average thickness of 6 µm (product name: Lumirror (registered trademark) #6C F531, manufactured by TORAY INDUSTRIES, INC.) serving as a substrate so that the average thickness of the applied ink was to be 10 µm, thereby producing an ink ribbon

### <Formation of Non-Flow Path Pattern 1>

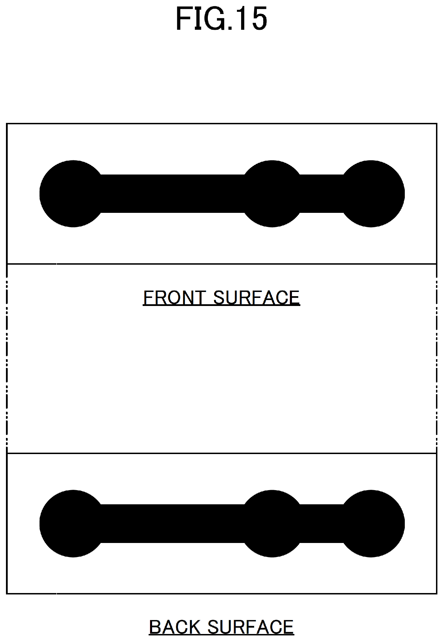

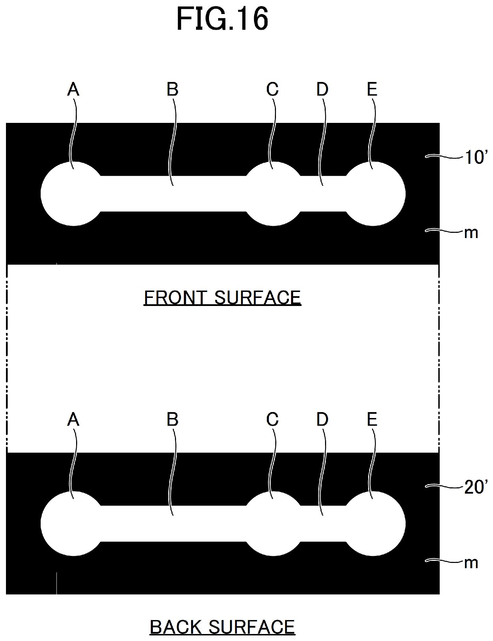

Flow Path Pattern 1 (a set of a flow path pattern for a front surface and a flow path pattern for a back surface) illustrated in Fig. 15 was printed on wood-free paper using a thermal transfer printer (device name: L'esprit R412v-ex, manufactured by SATO HOLDINGS CORPORATION) and the ink ribbon, thereby forming Non-Flow Path Pattern 1 (a set of a non-flow path pattern for a front surface and a non-flow path pattern for a back surface) that was a pattern corresponding to the printing portion of the ink ribbon from which Flow Path Pattern 1 was absent (see Fig. 16).

### <Formation of Non-Flow Path Pattern 2>

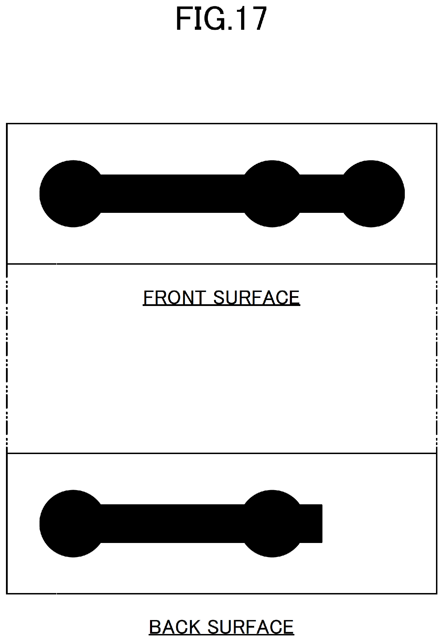

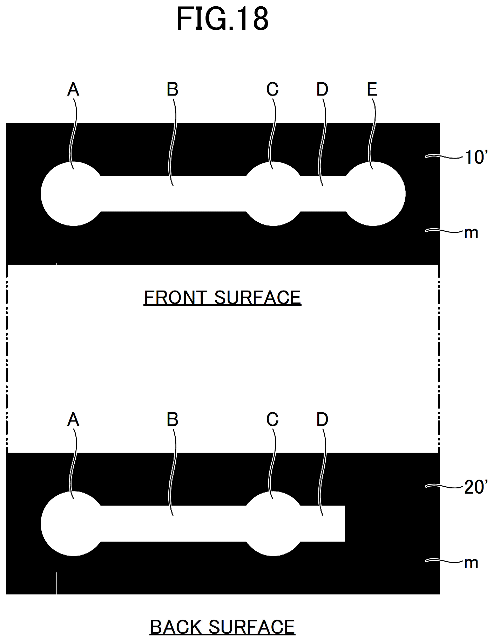

Flow Path Pattern 2 (a set of a flow path pattern for a front surface and a flow path pattern for a back surface) illustrated in Fig. 17 was printed on wood-free paper using a thermal transfer printer (device name: L'esprit R412v-ex, manufactured by SATO HOLDINGS CORPORATION) and the ink ribbon, thereby forming Non-Flow Path Pattern 2 (a set of a non-flow path pattern for a front surface and a non-flow path pattern for a back surface) that was a pattern corresponding to the printing portion of the ink ribbons from which Flow Path Pattern 1 was absent (see Fig. 18).

### (Example 1)

### <Formation of flow path>

The pieces of the ink ribbon having Non-Flow Path Pattern 1 illustrated in Fig. 16 were respectively fixed on a front surface and a back surface of filter paper (product name: Whatman (registered trademark) quantitative filter paper #41, manufactured by Global Life Sciences Technologies Japan K.K.), serving as a material M having a porous structure, and the wax ink was printed on the filter paper using a laminator (device name: GL535ML, manufactured by GBC).

The above stack was passed through the laminator set at a predetermined temperature to sequentially undergo the following laminating conditions (1) to (3) to allow the wax ink in the predetermined shape to penetrate from the front surface and the back surface of the filter paper to form a three-dimensional shape flow path in the filter paper, thereby producing an intermediate product of a sheet-like structure.

### -Laminating conditions-

(1) The above stack was passed through the laminator at the set temperature of 85°C at the line speed of 10 mm/sec, thereby transferring the ink to the filter paper.
(2) Subsequently, the resultant filter paper with the transferred ink was passed through the laminator at the set temperature of 85°C at the line speed of 5 mm/sec, thereby allowing the ink to penetrate the filter paper.
(3) Further, the resultant filter paper was passed through the laminator at the set temperature of 85°C at the line speed of 5 mm/sec, thereby allowing the ink to penetrate the filter paper.

### (Example 1)

### <Formation of support layer>

An adhesive tape (product name: 660PF, manufactured by NICHIBAN Co., Ltd.) serving as a support layer was stacked on the obtained intermediate product of the sheet-like structure to cover the back surface of the detection portion of the intermediate product of the sheet-like structure, and the resultant stack was laminated using a hand roller under a room temperature condition, thereby producing a sheet-like structure of Example 1, in which the support layer was formed on the back surface of the detection portion.

The sheet-like structure of Example 1 corresponds to the sheet-like structure of the first embodiment illustrated in Figs. 1 to 3.

As illustrated in Figs. 1 to 3, the sheet-like structure 11 of Example 1 had the porous structure layer 101 that included the flow path X having the porous structure through which a fluid could could pass and the non-flow path Y that did not allow the fluid to pass through. The flow path X included the fluid receiving portion A, the flow path portions B, C, and D, and the detection portion E, and the fluid receiving portion A, the flow path portions B, C, and D, and the detection portion E were connected in this order so that the fluid could pass through. In the fluid receiving portion A and the flow path portions B, C, and D, the front surface and the back surface of the porous structure layer 101 communicated with each other. The sheet-like structure 11 further included the support layer 301 disposed to cover the back surface of the detection portion E so that the porous structure layer was not exposed at the back surface of the detection portion E.

The configuration outline of the sheet-like structure is presented in Table 1.

### <Evaluations>

The capillary flow time evaluation and the color development unevenness evaluation for the detection portion were performed in the following manner. The evaluation results are presented in Table 2.

### <<Capillary flow time evaluation>>

The capillary flow time (CFT) refers to the time required for pure water to be soaked up to reach a 4 cm line when one end of a test piece, such as filter paper or the like, is placed upright in the pure water.

The capillary flow time (CFT_{f}) of the flow path portion, the capillary flow time (CFT_{d}) of the detection portion, and the ratio (CFT_{d}/CFT_{f}) of CFT_{d} to CFT_{f} were evaluated in the following manner.
(1) As a test piece, a sheet-like structure for testing having a strip shape in the size of 1 cm × 6 cm was prepared for each of the flow path portion and the detection portion.
   The sheet-like structure for testing for the flow path portion of the sheet-like structure of Example 1 was filter paper itself, and the sheet-like structure for testing for the detection portion of the sheet-like structure of Example 1 was a stack in which the support layer was stacked on the back surface of the filter paper.
(2) The time required for pure water to be soaked up to reach a 4 cm line of the sheet-like structure for testing from the time when the end of the sheet-like structure for testing was vertically immersed in the pure water was measured by a stopwatch.
(3) The measurement was performed with the sample number of n = 6, the average of the measurement values was calculated, and the obtained average value was determined as each of the capillary flow time (CFT_{f}) of the flow path portion, and the capillary flow time (CFT_{d}) of the detection portion. In addition, the ratio (CFT_{d}/CFT_{f}) of CFT_{d} to CFT_{f} was calculated.

In the case where the detection portion had the back surface barrier in Examples 2 to 4, the transfer and penetration of the wax ink were performed on the back surface of the filter paper, thereby preparing a sheet-like structure for testing for the detection portion in which the back surface of the detection portion was impregnated with the hydrophobic material to form a non-flow path portion. The evaluation was similarly performed in the processes of the above (1) to (3) using the prepared sheet-like structure for testing.

### <<Color development unevenness evaluation for detection portion>>

The evaluation of unevenness in the color development of the detection portion was performed in the following manner.
(1) An aqueous fluorescent ink (Fluorescent coat 96 Sky Color, manufactured by TOMBOW PENCIL CO., LTD.) in an amount of 1 µL was dropped on the flow path portion C using a micropipette.
(2) Subsequently, 12 µL of distilled water was dropped on the fluid receiving portion A.
(3) As the distilled water moved through the fluid receiving portion A, the flow path portions B, C, and D, and the detection portion E due to the capillary action, the aqueous fluorescent ink was moved to the detection portion E, together with the distilled water.
(4) Ten minutes after the dropping of the distilled water, an image of the surface of the detection portion E was captured by a scanner.
(5) Using image processing software Image J (https://imagej.net/ij/), the cyan color intensity was quantified from the cyan color information on the entire front surface of the detection portion E serving as a a target region, and an average value of the cyan color intensity values obtained with the evaluation sample number of n = 12 was determined as the "color development intensity."
(6) For each target region, the coefficient of variation (%) was calculated from the average value and the standard deviation of the cyan color distribution. The average value of the coefficient of variation obtained with the evaluation sample number of n = 12 was determined as the "color development unevenness." The smaller the value of the "color development unevenness" is, the smaller the color development unevenness is.

### (Comparative Example 1)

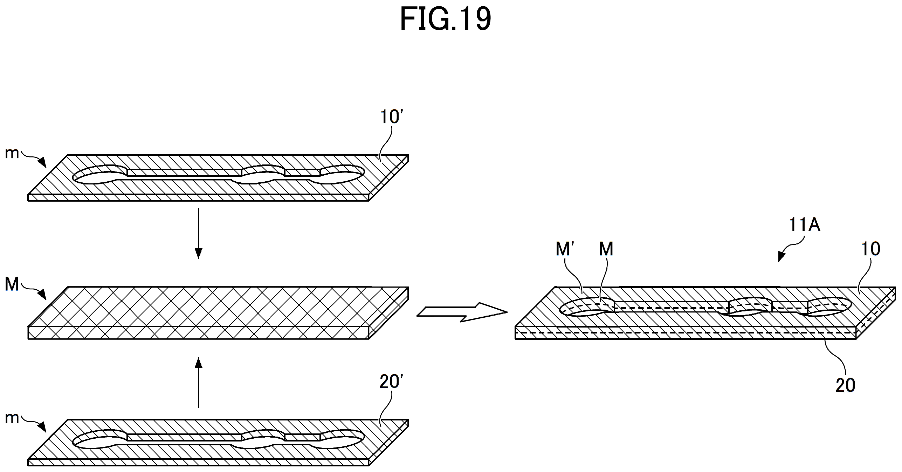

A sheet-like structure of Comparative Example 1 was produced and evaluated in the same manner as in Example 1, except that the support layer was not formed, and the intermediate product of the sheet-like structure was used as the sheet-like structure of Comparative Example 1. Fig. 19 illustrates a schematic plan view of the production process of the sheet-like structure of Comparative Example 1. The configuration and evaluation results of the sheet-like structure are presented in Tables 1 and 2.

### (Example 2)

A sheet-like structure of Example 2 was produced and evaluated in the same manner as in Example 1, except that a flow path was formed using Non-Flow Path Pattern 2 illustrated in Fig. 18 instead of Non-Flow Path Pattern 1 to provide the non-flow path on the back surface side of the detection portion, the support layer was not formed, and the average thickness of the wax ink for the back surface side of Non-Flow Path Pattern 2 was changed from 10 µm to 6 µm so that the thickness of the non-flow path at the back surface side of the detection portion was reduced and the ratio (t2/t1) was set to 6/10.

The configuration and evaluation results of the sheet-like structure are presented in Tables 1 and 2.

The sheet-like structure of Example 2 corresponds to the sheet-like structure of the third embodiment illustrated in Figs. 4, 5, and 7.

### (Example 3)

A sheet-like structure of Example 3 was produced and evaluated in the same manner as in Example 2, except that the average thickness of the wax ink for the back surface side of Non-Flow Path Pattern 2 was changed from 6 µm to 10 µm so that the thickness of the non-flow path at the back surface side of the detection portion was increased and the ratio (t2/t1) was set to 1/1. The configuration and evaluation results of the sheet-like structure are presented in Tables 1 and 2.

The sheet-like structure of Example 3 corresponds to the sheet-like structure of the second embodiment illustrated in Figs. 4 to 6.

### (Example 4)

A sheet-like structure of Example 4 was produced and evaluated in the same manner as in Example 3, except that a support layer was formed on the entire back surface of the sheet-like structure in the following manner. The configuration and evaluation results of the sheet-like structure are presented in Tables 1 and 2.

The sheet-like structure of Example 4 corresponds to the sheet-like structure of the fourth embodiment illustrated in Figs. 8 to 10.

### <Formation of support layer>

An adhesive tape (product name: 660PF, manufactured by NICHIBAN Co., Ltd.) serving as a support layer was stacked on the sheet-like structure of Example 3 to cover the entire back surface of the sheet-like structure, and the resultant stack was laminated using a hand roller under a room temperature condition, thereby producing the sheet-like structure of Example 4, in which the support layer was formed on the entire back surface of the sheet-like structure.

**[Table 1]**

| | Detection portion | Back surface of detection portion | Support layer | Wax ink thickness | |
|---|---|---|---|---|---|
| | | | | Front surface [µm] | Back surface [µm] |
| Comp. Ex. 1 | Communicating between front and back | Exposed | - | 10 | 10 |
| Ex. 1 | Communicating between front and back | Not exposed | Only back surface of detection portion | 10 | 10 |
| Ex. 2 | Back surface barrier* | Not exposed | - | 10 | 6 |
| Ex. 3 | Back surface barrier | Not exposed | - | 10 | 10 |
| Ex. 4 | Back surface barrier | Not exposed | Entire back surface | 10 | 10 |

| | | | | | |
|---|---|---|---|---|---|
| * "Back surface barrier" means that the porous structure at the back surface of the detection portion is impregnated with the hydrophobic material to form a non-flow path at the back surface side of the detection portion. | | | | | |

**[Table 2]**

| | CFT of flow path portion | CFT of detection portion | Ratio | Color intensity | Color development unevenness [%] |
|---|---|---|---|---|---|
| | CFTf [sec/4cm] | CFTd [sec/4cm] | CFT_{d}/CFT_{f} | | |
| Comp. Ex. 1 | 95.1 | 95.1 | 1.0 | 0.820 | 19.7 |
| Ex. 1 | 95.1 | 116.5 | 1.2 | 0.881 | 16.0 |
| Ex. 2 | 95.1 | 175.0 | 1.8 | 0.885 | 14.7 |
| Ex. 3 | 95.1 | 278.9 | 2.9 | 0.947 | 9.0 |
| Ex. 4 | 116.5 | 278.9 | 2.4 | 0.955 | 8.0 |

As is clear from Table 2, the capillary flow time (CFT_{f}) of the flow path portion of the sheet-like structure of Example 1 was 95.1 [sec/4 cm], the flow rate of the fluid was as high as 120 [sec/4 cm] or less, and rapidness of a reaction was maintained.

The capillary flow time (CFT_{d}) of the detection portion was 116.5 [sec/4 cm], which was 300 sec/4 cm or less and satisfied CFT_{f} < CFT_{d}. The ratio (CFT_{d}/CFT_{f}) of CFT_{d} to CFT_{f} was 1.2, which satisfied the favorable range of 1.2 or greater and 2.9 or less, and the color development unevenness was 16.0% (16.0% or less). Accordingly, it was found that the color development was evenly performed and the quantitative accuracy was improved compared to Comparative Example 1 in which the ratio (CFT_{d}/CFT_{f}) was 1.

In Examples 2 and 3, the capillary flow time (CFT_{d}) of the detection portion was prolonged, the ratio (CFT_{d}/CFT_{f}) was increased, and the color development unevenness was further reduced by providing the non-flow path at the back surface side of the detection portion. Accordingly, it was found that the color development was evenly performed and the quantitative accuracy was improved while maintaining rapidness of a reaction.

It was found that, in Example 4, the capillary flow time (CFT_{f}) of the flow path portion was prolonged by providing the support layer on the entire back surface of the porous structure layer, but the flow rate of the fluid was as fast as 120 [sec/4 cm] or less, and rapidness of a reaction was maintained.

This international application claims priority based on Japanese Patent Application No. 2023-217596 filed on December 25, 2023, and the entire contents of Japanese Patent Application No. 2023-217596 are incorporated herein by reference.

### REFERENCE SIGNS LIST

- 11: sheet-like structure of first embodiment
- 12: sheet-like structure of second embodiment
- 13: sheet-like structure of third embodiment
- 14: sheet-like structure of fourth embodiment
- 15: sheet-like structure of fifth embodiment
- 16: sheet-like structure of sixth embodiment
- 17: sheet-like structure of seventh embodiment
- 18: sheet-like structure of eighth embodiment
- 11A: sheet-like structure (Comparative Example 1)
- 101: porous structure layer
- 102: porous structure layer
- 103: porous structure layer
- 104: porous structure layer
- 301: support layer
- 304: support layer
- 306: support layer
- A: fluid receiving portion (flow path)
- B: flow path portion (flow path)
- C: flow path portion (flow path)
- D: flow path portion (flow path)
- E: detection portion (flow path)
- X: flow path
- Y: non-flow path
- m: hydrophobic material
- M: material
- M': material

## Claims

1. A sheet-like structure, comprising:
a porous structure layer including a flow path and a non-flow path, the flow path having a porous structure through which a fluid can pass, and the non-flow path not allowing the fluid to pass through,
wherein the flow path has a fluid receiving portion, a flow path portion, and a detection portion,
the fluid receiving portion, the flow path portion, and the detection portion are connected in this order so that the fluid can pass through, and
a ratio (CFT_{d}/CFT_{f}) of capillary flow time CFT_{d} of the detection portion to capillary flow time CFT_{f} of the flow path portion is 1.2 or greater.

2. The sheet-like structure according to claim 1,
wherein the ratio (CFT_{d}/CFT_{f}) of the capillary flow time CFT_{d} of the detection portion to the capillary flow time CFT_{f} of the flow path portion is 1.2 or greater and 2.9 or less.

3. The sheet-like structure according to any one of claims 1 to 3,
wherein the capillary flow time CFT_{f} of the flow path portion is 120 sec/4 cm or less.

4. The sheet-like structure according to any one of claims 1 to 4,
wherein the capillary flow time CFT_{d} of the detection portion is 300 sec/4 cm or less.

5. The sheet-like structure according to any one of claims 1 to 5,
wherein the non-flow path is the porous structure impregnated with a hydrophobic material.

6. The sheet-like structure according to any one of claims 1 to 6,
wherein the flow path includes the fluid receiving portion and the flow path portions, which communicate with a first surface and a second surface of the porous structure layer, and the detection portion that communicates with the first surface but is not exposed at the second surface.

7. The sheet-like structure according to claim 6,
wherein the sheet-like structure further comprises a support layer on the second surface of the detection portion, or
the porous structure at the second surface of the detection portion is impregnated with a hydrophobic material.

8. The sheet-like structure according to claim 3 or 6, further comprising:
a support layer on the second surface of the fluid receiving portion and the flow path portion.

9. The sheet-like structure according to claim 8,
wherein the support layer is impermeable to water.

10. The sheet-like structure according to any one of claims 1 to 9,
wherein the sheet-like structure is an inspection device.
